# EUROPEAN PATENT APPLICATION

(11) **EP 1 496 481 A2**
(43) Date of publication of application: **12.01.2005**
(21) Application number: 04015958.4
(22) Date of filing: 07.07.2004
(51) Int. Cl.: G07F 11/00, G06F 19/00

(54) **Unit for remote pharmacological assistance**

(30) Priority: 10.07.2003 IT TO20030535
(71) Applicant: L'UNIONE DI AMULIO GUBBINI, 10080 San Benigno Canavese(TO) (IT)
(72) Inventor: Gubbini, Amulio, 10080 San Benigno Canavese (TO) (IT)
(74) Representative: Lotti, Giorgio

(57) **Abstract**

Described herein is a unit (1) for remote pharmacological assistance provided with: an automatic vending machine (2) of products (3) of a pharmaceutical nature; a device for recognition, at input to and at output from the vending machine (2), of the products (3); and a control device associated to the recognition device for control of the automatic vending machine (2); the control device having: an interface for interfacing the automatic vending machine (2) with a user of the automatic vending machine (2) itself; a device for exchange of information with the user; and a connection of the automatic vending machine (2) to a central network for replenishment of the vending machine (2) itself and for connection of the user with a call centre.

## Description

The present invention relates to a unit for remote pharmacological assistance.

In general, known in the field of pharmacological assistance are automatic units for distribution of medicinal preparations comprising a show case for exhibiting to a user the type of drugs contained therein, a selection display to enable the user to select the desired drug, and a device designed to supply the drug selected at output from the unit itself.

Automatic units of the type described above are normally installed in the proximity of chemist's, and manifest their drawbacks above all during the normal closing hours of chemist's shops, in so far as the user is no longer provided with the possible assistance of the personnel of the chemist's itself, and is forced to make substantially mechanical choices irrespective of his own real needs.

The purpose of the present invention is to provide a unit for remote pharmacological assistance which is not only free from the drawbacks described above but may also be installed in centres of population that are without chemist's shops.

According to the present invention a unit for remote pharmacological assistance is provided comprising:
- an automatic vending machine for dispensing products of a pharmaceutical nature, the vending machine being provided with a magazine, an outlet, and a device for picking up the products, which is set between the magazine and the outlet itself;
- means for recognition of the products at input to and at output from the vending machine; and
- control means associated to the recognition means for controlling the automatic vending machine;
the unit being characterized in that the control means in turn comprise:
- interface means for interfacing the automatic vending machine with a user of the automatic vending machine itself;
- means for exchanging information with said user; and
- means for connection of the automatic vending machine to a central network for supply of said magazine and for connection of said user with a centre of assistance.

The invention will now be described with reference to the annexed drawings, which illustrate a non-limiting example of embodiment thereof, and in which:

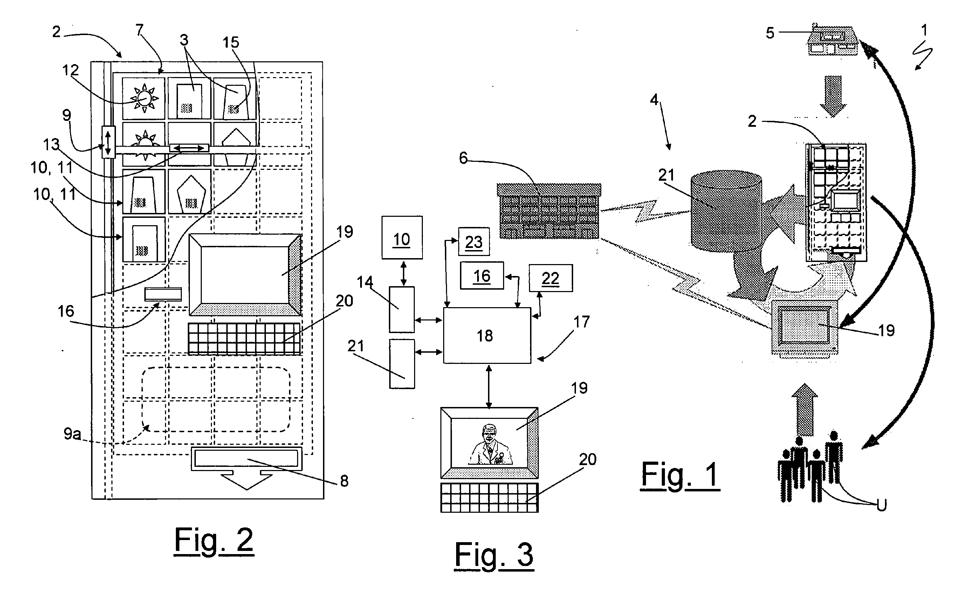

- Figure 1 is a schematic view of a preferred embodiment of a unit for remote pharmacological assistance according to the present invention;
- Figure 2 is a schematic view, in front elevation and at an enlarged scale, of a detail of Figure 1;
- Figure 3 is a schematic view, with parts removed for reasons of clarity, of a detail of the unit of Figure 2; and
- Figures 4, 5a, 5b and 6 are block diagrams illustrating schematically operation of the unit of Figure 1.

With reference to the annexed figures, the reference number 1 designates as a whole a unit for remote pharmacological assistance.

The unit 1 comprises an automatic vending machine 2 for dispensing products 3 of a pharmaceutical type, and a network 4 for connection of the automatic vending machine 2 both to a provisioning centre 5 for supply and maintenance of the vending machine 2 and to a centre 6 of assistance, said network enabling connection of a user U of the unit 1 itself to a call centre.

The vending machine 2 comprises a magazine 7, an outlet 8 for the products 3, a device 9 for picking up the products 3 themselves, which is set between the magazine 7 and the outlet 8 itself, and a refrigerating device 9a designed to refrigerate the products 3 that are arranged inside the magazine 7.

In particular, the magazine 7 comprises a plurality of compartments 10 arranged parallel to one another in rows and columns and each having a front area 11, which functions both as a respective loading inlet and as a respective supply outlet, and for each container a device 12 for feeding the products 3 from the loading inlet towards the corresponding supply outlet.

The device 9 for picking up comprises a manipulator 13, which is mobile at the front at the front areas 11 and is designed to receive the products 3 from the compartments 10 for conveying them to the outlet 8, preventing any damage to the products 3 themselves.

The vending machine 2 further comprises a barcode-reading device 14, which is designed to scan at input to and at output from the vending machine 2 itself the barcodes 15 of the products 3 so as to keep under control certain parameters such as, above all, the amount of products 3 contained in the magazine 7 and an expiry date D of the products 3 themselves. The reading device 14 is moreover interfaced with the compartments 10 of the magazine 7, and is designed to direct a person responsible for replenishing the magazine 7 itself to the compartment 10 suitable for the type of product 3 during loading so as to reduce, if not altogether rule out, any storage errors that would lead to supply of products 3 not required according to the specifications of the user U.

The vending machine 2 further comprises a device 16 for recognition and collection, designed to recognize possibly the user U and to collect the amount due from the user U in relation to the products 3 to be withdrawn either via introduction of money or via introduction of a credit card, or else by means of personalized cards, the so-called "smart cards", which comprise an integrated circuit bearing the personal data and the pathological data of the user U as well as storing the amount of products 3 withdrawn by the user U from the vending machine 2 or from other vending machines 2 located in other points of the territory. In fact, since the vending machines 2 are connected by means of the network 4 for connection to a centre 6 it is possible to monitor the withdrawals made by each individual user U, thus preventing any possible overdosing and guaranteeing a higher degree of safety for the user U himself. Furthermore, the use of personalized cards enables not only drawing-up of anamnestic-data sheets and statistics regarding the user U, but also the creation of a data base, for example, at the Ministry of Health, in order to monitor at a national level the amount of drugs used.

The unit 1 further comprises an information unit 17 integrated in the vending machine 2, which is not only designed to control all the functions of the vending machine 2 itself, but is also capable of interacting with the user U to help him in the choice of the product 3 that is most appropriate, thus rendering the unit 1 suitable for installation in centres of population that are substantially isolated and without any local pharmaceutical assistance.

The information unit 17 comprises a computerized control device 18 associated to the device 14, a display 19, and an alpha-numeric keypad 20 connected both to the device 18 for interfacing the vending machine 2 with a user U and for exchanging information with the user U himself.

The control device 18, which substantially constitutes the core of the unit 1 according to the invention, operates on the basis of a software program provided for processing the results necessary in relation to the requests of the user U, for controlling the state of the supplies in the magazine 7, and for calculating the aforesaid amount due from the user U, and interacts with a data bank 21, which comprises the data of the users U, the amount of products 3 withdrawn by each individual user U in the case of use of personalized cards, and the data of the products 3 present in the magazine 7. All these data can be used in statistical terms, enabling an optimal management of the unit 1 both in terms of maintenance and in terms of provisioning of the amounts and types of products 3.

The data bank 21 also comprises a database of the SQL Server type, which enables storage of the data regarding the drugs present on the market, and is in turn connected to the centre of assistance 6 by means of a TeSys telemetering service, which enables immediate notification of the assistance operators at the onset of any possible malfunctioning.

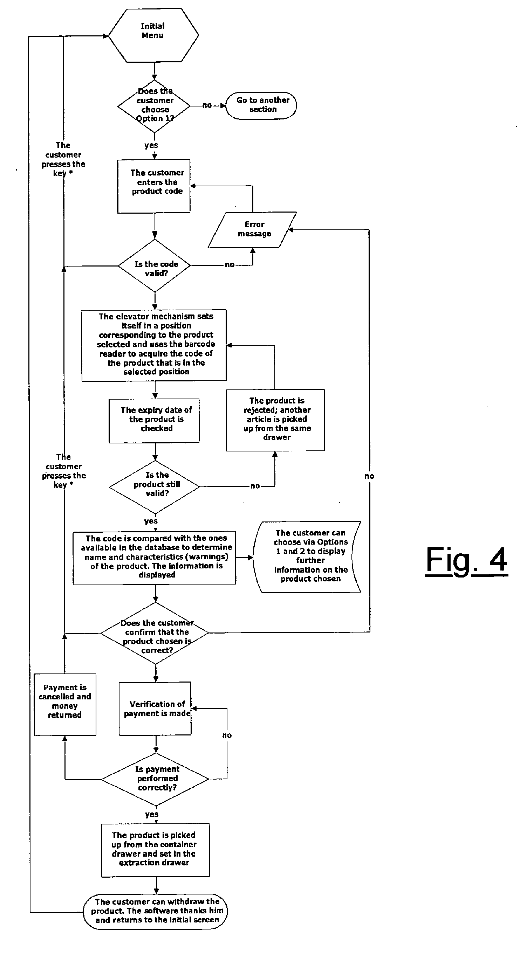

The logic operation of the unit 1 referred to above will now be described with initial reference to the block diagram illustrated in Figure 4 and corresponding to the case where the user U cannot be identified by the unit 1 itself.

Upon activation of the information unit 17 by pressing of the key "*" on the keypad 20, the display 19 will show a welcome message for the choice of the language to use, and, once said choice has been made via the keypad 20, an initial menu will come up with a message consisting of the question "What do you want to do?" and the possible answer options to said question, namely:
1. Purchase product
2. Ask for advice
3. Connect up to Operator
4. Ask for Information
5. Get information on the vending machine

By pressing, on the keypad 20, the key corresponding to the option selected, the user enters the desired environment.

At any moment it will be possible to change the language in which all the messages are displayed, without the need to return to the initial menu, by simply pressing the key "*", and at any moment it will be possible to return to the main menu by simply pressing the key "#".

Starting from the initial menu, the user U can choose different modes of operation of the vending machine 2, and the reason for the multiple interface is to enable the user U who already knows the product 3 to be withdrawn to reduce the time of occupation of the vending machine 2 by activating a procedure of "direct" purchase of the products 3.

The products 3 available are all present in the magazine 7 and are all identified by a respective numerical product code C, which defines the compartment 10 in which the products 3 are located. Entry of the code C, which is done by simply typing it on the keypad 20, activates a procedure that leads to identification and verification of the product 3 itself, to payment, and to delivery of the product 3 at the outlet 8.

Once the product 3 has been chosen, the device 14 that detects the barcode 15 on the product 3 is activated and uses this code 15, and not the code that can be inferred from the position of the product 3 in the magazine 7, for its identification and for verification on the part of the user U that the product 3 selected is correct. Likewise, a check of the expiry date of the product 3 is performed.

Once the product 3 has been identified, there immediately appears on the display 19 both the fundamental information, which can for example be entered into the data bank 21 by a person responsible for running and administering the vending machine 2, and which gives the name of the product 3, its price, and the description of the product 3 itself, and some accessory information, such as warnings, dosage, and possible contra-indications for a correct use of the product 3 itself.

If the product 3 is confirmed, the user U may proceed by means of the device 16 to paying the amount due, and may withdraw the product 3 from the outlet 8, in which the product 3 will have been deposited by the device 9.

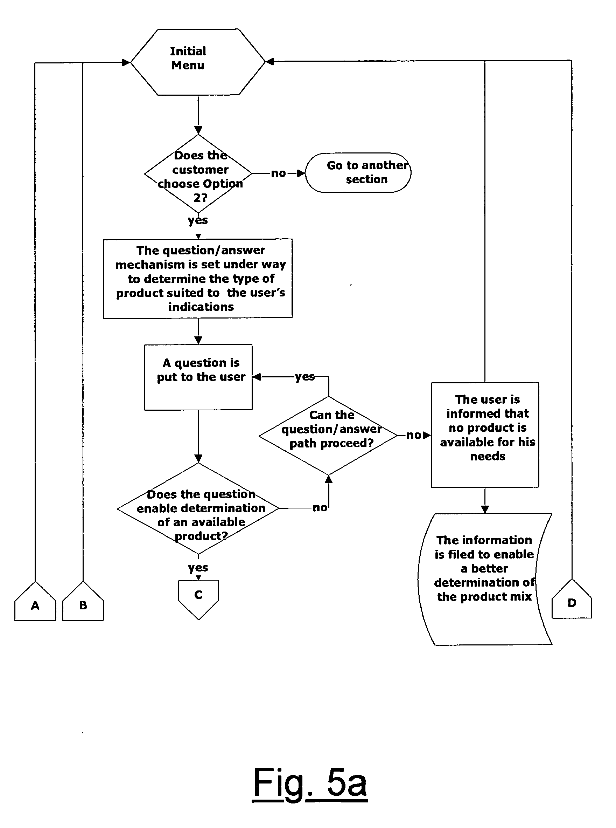

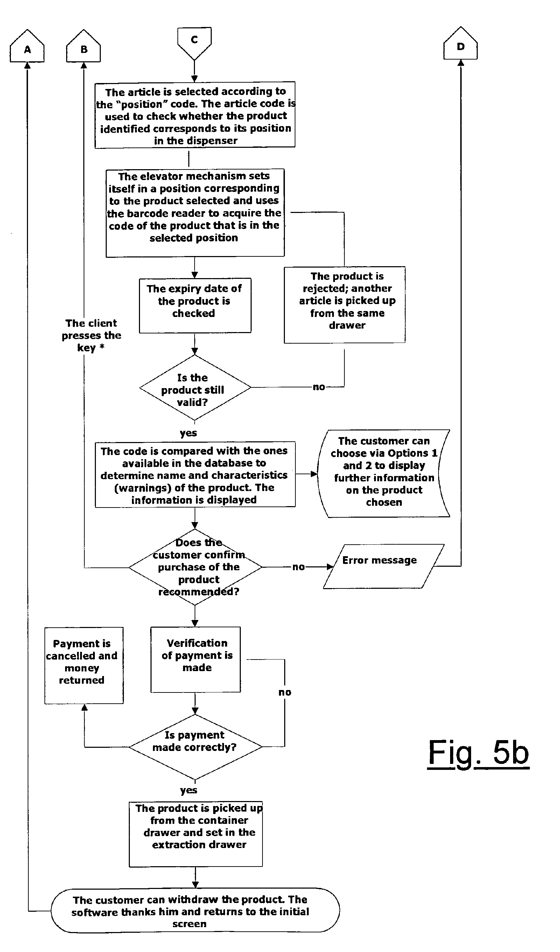

In the case where the user U wishes to receive advice regarding the type of product 3 to use in the case of onset of given symptoms or pathological conditions, the user U must choose from the main menu the procedure corresponding to "request for advice", illustrated in the block diagrams of Figures 5a and 5b.

In this case, the information unit 17 proposes, on the display 19, a series of simple questions according to a prearranged path that guides the user U step by step to a correct definition of the his own needs and determines, according to the answers given by the user U, which, amongst the products 3 available in the magazine 7, is the product 3 best indicated for the problem of the user U.

In the case where it is not possible to create a path that leads to one of the products 3 immediately available in the vending machine 2, in the first place the user U receives either a suggestion on a product even if this product is not available in the vending machine 2 or else advice adequate for the type of symptoms encountered. In any case, the information entered by the user U will be stored in the data bank 21 and may be used for drawing up statistics that can be used by the administrator G of the vending machine 2 itself for making up in the most adequate way possible the mix of the products 3 available.

In the case where, instead, a product 3 corresponding to the needs of the user U is available, the process proceeds along the lines of what has been described previously in regard to a direct purchase of the product 3.

In this case, however, the device 14 controls whether the position in which the products 3 chosen are stored coincides with what is declared during loading of the compartments 10. In other words, the code C corresponding to the product 3 recommended is compared with the code 15 and, if the two codes C and 15 coincide, then the product 3, which evidently is in the correct position, is delivered to the user U.

In the case where the user U needs, instead, direct assistance, he must choose from the main menu the procedure corresponding to "connection to an operator" or "call centre".

In this case, the information unit 17 sets the users U directly in connection with a specific call centre capable of round-the-clock supply of adequate information. The type of connection hypothesised is based upon the Internet for those situations in which a connection of the "flat" type is available so as to reduce considerably the running costs of the service, or else transmission of information is obtained vocally in so far as the vending machine 2 can be connected also to a telephone line. Alternatively, once again, for those situations in which a connection of the ADSL type is available, transmission of information comes about in video-conference between the user U, framed by a telecamera 22 forming part of the information unit 17, and an operator of the call centre.

Once in contact with the call centre in the ways described above, the user U can also then be put in contact with a qualified chemist who is able to solve the user's problem in the best possible way.

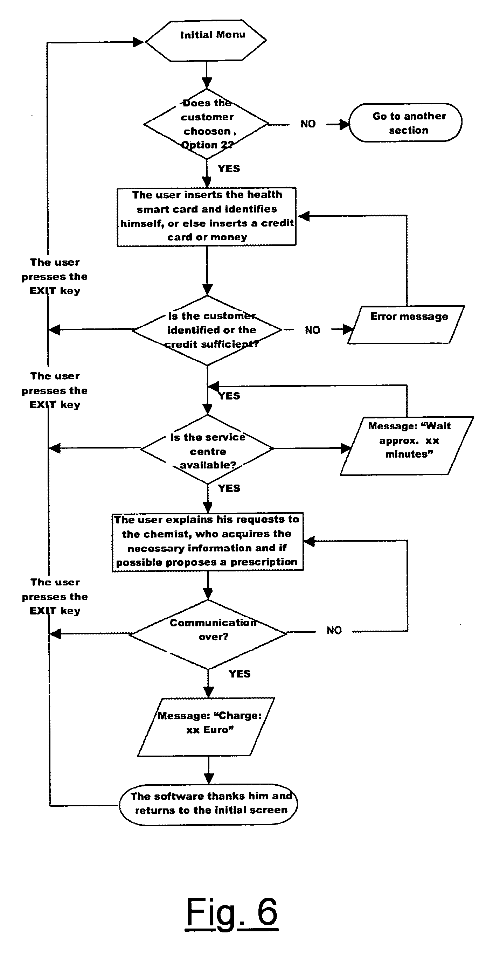

In the case where the user U can be identified by the unit 1 itself using a personalized card of the "smart card" type and can therefore be recognized by the unit 1 itself by means of an identifying code, the logic operation of the unit 1 can be described with reference to the block diagram illustrated in Figure 6.

In this case, the user U, in addition to the options described above, can get directly in touch with an operator of the call centre, who will not only possess adequate information on the "history" of the user U, as well as, above all, on the relevant pharmaceutical products associated to the user, but will also be able to prescribe particular drugs to the user U himself after having heard the specific needs of the user U.

The adoption, for the unit 1, of a number of vending machines 2 interconnected to the network 4, and conveying to a data bank 21 of all the information exchanged with the users U via the information units 17 of each vending machine 2 via connection to the Internet not only enables remote management of the vending machines 2 themselves but also enables recording of all the data corresponding to the amounts and modes of consumption of the products 3 of each vending machine 2.

The information gathered will come to constitute a thoroughgoing observatory on the modes of use of the pharmaceutical products 3 that may be sold with the vending machines 2 described above.

It will moreover be possible to manage the magazines 7 as peripherals of a single central magazine and obtain a considerable overall efficiency of operation thereof as regards, for example, the state of the supplies and the statistics of use.

More simply, it will be possible to create local networks formed by a number of vending machines 2 serving a single chemist's both in terms of data communication and in terms of management of the supplies. This solution can be useful for meeting the needs, for example, of mountain communities or else of rural communities that are without chemist's and are isolated from the major centres of population. In this case, the municipalities of said communities will be able to make available a protected open place, and the chemist's of the closest centre of population may install therein a satellite vending machine 2 connected with the vending machine 2 of the chemist's itself.

Furthermore, in the case where the user U needs simply information regarding chemist's shops that are scheduled to be open, useful numbers, timetables, services, etc., the user U will have to choose from the main menu the procedure corresponding to the "request for information", and the information unit 17 will guide the user U himself to the answers that he needs via a series of options that can appear on the display 19 and can be selected by means of the keypad 20.

By accessing the option "Get information on vending machine" from the main menu, the user U will be able to receive information on the vending machine 2 and the unit 1. Some of this information can be rendered accessible only to the licensee of the vending machine 2, who, in order to display said information, will have to enter a password for access.

Finally, in the case (which is not illustrated in detail but can be readily inferred from what has been described above) where the vending machine 2 is connected to a chemist's provided with an automatic stores, the vending machine 2 comprises a device 23 for automatic reading of the medical prescriptions, which is connected to the device 18 and comprises, in turn, a scanner of a known type, and an OCR (Optical Character Reading) reader, which is also of a known type.

The device 23 enables reading of the medical prescriptions identifying both the user U and the prescribed drugs that he needs.

Automatic reading of medical prescriptions will enable distribution of prescribed drugs also when the chemist's are closed, so making for a more widespread dissemination over the territory.

It is understood that the invention is not limited to the embodiments described and illustrated herein, which are to be considered merely as examples of embodiment of the unit for remote pharmacological assistance, which may instead undergo further modifications corresponding to shapes and arrangements of parts, and details of construction and assembly.

## Claims

1. A unit (1) for remote pharmacological assistance comprising:
- an automatic vending machine (2) for dispensing products (3) of a pharmaceutical nature, the vending machine (2) being provided with: a magazine (7); an outlet (8); and a device (9) for picking up the products (3), set between the magazine (7) and the outlet (8) itself;
- recognition means (14), at input to and at output from the vending machine (2), for recognizing the products (3); and
- control means (18) associated to the recognition means (14) for control of the automatic vending machine (2);
the unit (1) being **characterized in that** the control means (18) in turn comprise:
- interface means (19, 20) for interfacing the automatic vending machine (2) with a user (U) of the automatic vending machine (2) itself;
- means (19, 20, 21) for exchange of information with said user (U);
- means of connection (4) of the automatic vending machine (2) to a central network (4) for supply of said magazine (7) and for connection of said user (U) with a centre of assistance (6).

2. The unit according to Claim 1, **characterized in that** said recognition means (14) comprise a barcode-reading device (14) designed for scanning the products (3) at input to and at output from the vending machine (2), each product (3) being provided with a respective barcode (15) and a respective expiry date (D), which can be read by said recognition means (14).

3. The unit according to Claim 2, **characterized in that** the magazine (7) comprises a plurality of compartments (10), each of which is provided with a respective loading inlet (11) and a respective supply outlet (11); said recognition means (14) indicating the loading inlet according to the data scanned by the reading device (14).

4. The unit according to Claim 3, **characterized in that** the picking-up device (9) comprises a manipulator (13), which is mobile at the front at the outlets (11), and is designed to receive the products (3) from the compartments (10) for conveying the products (3) to the outlet (8) of the vending machine (2), preventing any damage to the products (3) themselves; the products (3) in the magazine (7) being identified by a respective numerical product code (C) defining the compartment (10) for positioning the corresponding product (3).

5. The unit according to Claim 1, **characterized in that** it comprises a device (16) for recognition and collection associated to the vending machine (2) and connected to said control means (18) for recognizing the user (U) and for collecting an amount due from the user (U) himself in relation to the products (3) to be picked up.

6. The unit according to Claim 5, **characterized in that** the device (16) for recognition and collection is designed to read personalized cards bearing the pathological data of the user U and the products (3) withdrawn by the user (U) from the vending machine (2) or from other vending machines (2) located in other points of the territory and forming part of the unit (1).

7. The unit according to Claim 6, **characterized in that** it comprises a data bank (21) for gathering data corresponding to the products (3) and data corresponding to the users (U).

8. The unit according to Claim 7, **characterized in that** it comprises a centre of assistance (6) connected with the data bank (21) for monitoring the withdrawals made by each individual user (U) and for providing anamnestic-data cards and statistics regarding the user (U) himself, preventing possible overdosing and guaranteeing a greater degree of safety for the user (U).

9. The unit according to any one of Claims 6 to 8, **characterized in that** said personalized cards are smart cards.

10. The unit according to any one of the preceding claims, **characterized in that** it comprises an information unit (17), which is integrated in the vending machine (2) and includes said control means (18) and said means for interface and exchange of information (19, 20, 21), the information unit (17) being designed to control all the functions of the vending machine (2) itself and to interact with the user (U) to help the user (U) himself in the choice of the most appropriate product (3), rendering the unit (1) suitable for installation in premises that are substantially isolated and without any local pharmaceutical assistance.

11. The unit according to Claim 10, **characterized in that** the information unit (17) operates on the basis of a computer program stored locally in the control means (18) or else peripherally and is accessible via said connection means (4).

12. The unit according to Claim 11, **characterized in that** the information unit (17) can be exploited by a user (U) via said means (19, 20, 21) for interface and exchange of information, and is designed to enable a user (U) who already knows the product (3) to be withdrawn to select directly said product code (C) for activating a procedure of direct purchase of the products (3), thus reducing the time of occupation of the vending machine (2).

13. The unit according to Claim 11, **characterized in that** the information unit (17) can be exploited by a user (U) by means of said means (19, 20, 21) for interface and exchange of information, and is designed to supply the user (U) with advice regarding the type of product (3) to use according to a definition of the symptoms or pathological conditions detected by the information unit (17) by getting the user (U) to follow a prearranged path of questions and answers.

14. The unit according to Claim 13, **characterized in that** said data bank (21) is designed to store the information gathered along said path of questions and answers for drawing up statistics that can be used for making up a mix of the products (3) that can be stored in the magazine (7).

15. The unit according to Claim 14, **characterized in that** said path of questions and answers is irrespective of the products (3) contained in the magazine (7); said interface means (19, 20) comprising a display (19) for displaying suggestions for the user (U) regarding products that cannot be withdrawn from the vending machine (2), or else regarding the type of symptoms encountered.

16. The unit according to Claim 12 or Claim 14, **characterized in that** said interface means (19, 20) comprise a display (19) for displaying, once the product (3) has been chosen, fundamental information contained in said data bank (21) regarding a name, a price, and a description of the product (3) selected, and, possibly, accessory information regarding warnings, dosage, and contra-indications of the product (3) selected.

17. The unit according to Claim 16, **characterized in that** said recognition means (14) are designed to detect the barcode (15) on the product (3) selected and to use this barcode (15) for identification and control of the expiry date (D) of the product (3) selected, and for activation of the picking-up device (9).

18. The unit according to Claim 11, **characterized in that** the information unit (17) can be exploited by a user (U) via said means (19, 20, 21) for interface and exchange of information, and is designed to provide assistance aimed at the user (U) by connecting the user (U) himself with a centre of assistance (6).

19. The unit according to any one of the preceding claims, **characterized in that** it comprises a device (23) for automatic reading of medical prescriptions, said device (23) in turn comprising means for reading the medical prescriptions and means for recognition of the medical prescriptions themselves.

20. The unit according to any one of the preceding claims, **characterized in that** said vending machine (2) comprises a refrigerating device (9a) for refrigerating products (3) arranged within the magazine (7).
